Europäisches Patentamt

European Patent Office

Office européen des brevets

(11) Veröffentlichungsnummer: **0 367 142**
**A2**

## (12) EUROPÄISCHE PATENTANMELDUNG

(21) Anmeldenummer: 89120005.7

(22) Anmeldetag: 27.10.89

(51) Int. Cl.5: **C08J 5/00 , C12N 11/00**

(30) Priorität: **29.10.88 DE 3836894**

(43) Veröffentlichungstag der Anmeldung:
**09.05.90 Patentblatt 90/19**

(84) Benannte Vertragsstaaten:
**AT BE CH DE ES FR GB IT LI NL**

(71) Anmelder: **KRC Umwelttechnik GmbH**
**Alfred-Nobel-Strasse 20**
**D-8700 Würzburg 1(DE)**

(72) Erfinder: **Maassen, Jürgen**
**Ravensburgstrasse 3**
**D-8707 Veitshöchheim(DE)**
Erfinder: **Wildenauer, Franz**
**Hamburgerstrasse 181**
**D-2800 Bremen 1(DE)**
Erfinder: **Kück, Werner**
**Vor dem Steintor 216**
**D-2800 Bremen 1(DE)**

(74) Vertreter: **Kaiser, Henning**
**SALZGITTER AG Patente und Lizenzen**
**Kurfürstendamm 32 Postfach 15 06 27**
**D-1000 Berlin 15(DE)**

(54) **Verfahren und Vorrichtung zum Herstellen von Biokatalysatoren umfassenden Perlen aus Perlen bildenden Lösungen.**

(57) Verfahren und Vorrichtung zum Herstellen von Perlen gleichen Durchmessers aus Perlen bildenden Lösungen, insbesondere hochviskosen Polymerlösungen, mit im Innern der Perlen immobilisierten Zellen oder Enzymen, bei denen die die Zellen oder Enzyme enthaltende Lösung durch Düsen gedrückt, der aus den Düsen austretende Strahl mittels Vibration in Tropfen zerlegt wird und die Tropfen in eine Härtungslösung fallen. Die Polymerlösung wird jeder einzelnen von mehreren, an einem mit einem Vibrator gekoppelten Düsenträger befestigten Düsen dosiert zugeführt, so daß sich gleichmäßige Perlen industriell erzeugen lassen.

## Verfahren und Vorrichtung zum Herstellen von Biokatalysatoren umfassenden Perlen aus Perlen bildenden Lösungen

Die Erfindung betrifft ein Verfahren und eine Vorrichtung zum Herstellen von Biokatalysatoren umfassenden Perlen gleichen Durchmessers aus Perlen bildenden Lösungen, insbesondere aus hochviskosen Polymerlösungen, mit im Innern der Perlen immobilisierten Zellen oder Enzymen, bei dem eine die Zellen oder Enzyme enthaltende Lösung durch Düsen gedrückt, der aus den Düsen austretende Strahl mittels Vibration in Tropfen zerlegt wird und die Tropfen in eine Härtungslösung fallen.

Unter Biokatalysatoren sind biologisch aktive Stoffe zu verstehen, die nach diesem Verfahren in perlenförmigen Körpern immobilisiert werden können sowie auch diese Körper selbst. Zum Herstellen von Biokatalysatoren werden Mikroorganismen, Zellen oder Enzyme einem wasserlöslichen, hochviskosen Polymer, wie z. B. Alginat oder Chitosan zugemischt. Durch Eintropfen in eine Vernetzungslösung werden die Zellen oder Enzyme im Inneren einer Perle immobilisiert. Die so gewonnenen Biokatalysatoren lassen sich in zahlreichen biotechnischen Prozessen einsetzen. Es wird dabei angestrebt, Perlen von möglichst gleichem Durchmesser zu verwenden.

Im Labormaßstab wird die hochviskose Lösung durch Kapillaren getropft und der Tropfen durch einen Luftstrom abgeblasen. Es liegt auf der Hand, daß mit diesem Verfahren nur kleine Mengen Perlen erzeugt werden können, die für eine technische Anwendung zu gering sind. Hinzu kommt, daß der Luftdurchsatz hoch und der erzielbare Durchmesser auf 1 bis 1,2 mm beschränkt ist.

Es wurde schon ein Verfahren vorgeschlagen, bei dem die Tropfen durch Abschleudern der Lösung von einem Drehteller erzeugt werden. Hierbei ist der Perlendurchmesser jedoch sehr ungleichmäßig.

Schließlich ist es auch bekannt, die Polymerlösung selbst durch einen vibrierenden Stößel oder eine vibrierende Membran zum Schwingen zu bringen, so daß die durch eine Kapillare gedrückte Lösung beim Austritt in Tropfen zerlegt wird.

Mit diesem Verfahren läßt sich der Tropfendurchmesser bei guter Reproduzierbarkeit vergrößern, jedoch müssen, will man hohe Durchsätze erzielen, mehrere Düsen beaufschlagt werden. Dies müßte äußerst gleichmäßig geschehen, was jedoch wegen Reflexionen und Resonanzen der Schwingungen in der Flüssigkeit zu Ungleichmäßigkeiten bei der Tropfenform und dem Durchmesser führt, so daß sich jeweils nur eine sehr kleine Anzahl von Kapillaren mit einem vibrierenden Stößel oder einer vibrierenden Membran beaufschlagen läßt.

Der Erfindung liegt daher die Aufgabe zugrunde, ein Verfahren zu schaffen, mit dem sich eine große Anzahl nach Form und Größe gleichmäßiger Perlen industriell herstellen läßt.

Gelöst wird die Aufgabe bei einem Verfahren der eingangs erwähnten Art dadurch, daß erfindungsgemäß die Lösung, beispielsweise eine Polymerlösung, jeder einzelnen von mehreren, an einem mit einem Vibrator gekoppelten Düsenträger angeordneten Düsen dosiert zugeführt wird. Jede Düse besitzt eine eigene Zuleitung, in der ein Dosierventil angeordnet ist, um den Zufluß individuell zu dosieren. Die Tropfengröße und Tropfenform läßt sich daher unabhängig von überlagerten Resonanzen und Reflexionen sowie unterschiedlichen Bedingungen in den Zuleitungen einstellen, so daß eine Vielzahl von Düsen an einem Düsenträger angeordnet sein kann und der Einsatz eines Vibrators zur Erzeugung gleicher Perlen im technischen Maßstab möglich ist.

Der Durchsatz, die Tropfengröße und die Tropfenform lassen sich durch Verändern der Vibrationsfrequenz, der Vibratoramplitude, des Drucks der Lösung und des Durchflusses durch die Dosierventile einstellen, so daß eine große Anzahl von Variablen gegeben ist, mit Hilfe derer sich die gewünschten Werte erreichen lassen und sowohl einzelne Düsen gesondert als auch alle Düsen gemeinsam beeinflußbar sind.

Eine Vorrichtung zur Durchführung des Verfahrens ist gekennzeichnet durch einen Druckvorlagebehälter, von dem die Lösung zu einem nachgeschalteten Verteilerblock gelangt. Mit diesem Verteilerblock sind Feindosierventile verbunden, von denen Leitungen zu mehreren, an einem Düsenträger angeordneten Düsen führen, die mittels eines starr mit dem Düsenträger gekoppelten Vibrators in Schwingungen versetzt werden. Besonders geeignet sind kegelförmige Düsen mit scharfer geschliffener Kante und einem Durchmesser von 0,1 bis 1,5 mm. Der Durchmesser ist nach den Eigenschaften der Lösung und der Tröpfchengröße sowie dem Durchsatz zu wählen.

Die Vorrichtung zum Herstellen der Perlen kann aus mindestens einem Ansatzbehälter für mit Zellen oder Enzymen gemischte Lösung, einer Filtrationsstrecke, wenigstens einem Druckvorlagebehälter, einem Verteilerblock, Feindosierventilen, einem Düsenträger, mehreren Düsen, einem Vibrator, einer unterhalb der Düsen angeordneten, mit Härtungslösung gefüllten Auffangrinne für die Perlen und wenigstens einem mit Härtungslösung gefüllten Härtungsbehälter bestehen. Auf diese Weise läßt sich im etwa kontinuierlichen Durchlauf und im industriellen Maßstab eine große Anzahl von Perlen mit definiertem Durchmesser, ausgehend von den einzelnen Bestandteilen wie Alginat bzw. Chitosan sowie den Zellen bzw. Enzymen herstellen.

Der Ansatzbehälter weist vorzugsweise ein Rührwerk, einen Dispergator und eine Einrichtung zur Einstellung und Beeinflussung der Temperatur der Lösung auf. Eine Heizvorrichtung kann aus einem in die Lösung eintauchenden Heizelement oder vorzugsweise aus einem den Ansatzbehälter umgebenden Heizmantel bestehen. Ein solcher Heizmantel weist den Vorzug auf, daß er sich wahlweise mit einer Heiz- oder einer Kühlflüssigkeit beaufschlagen läßt, so daß sich die Lösung im Ansatzbehälter sowohl erhitzen als auch kühlen läßt. Diese läßt sich nach einer bevorzugten Ausführung auch dadurch erhitzen oder abkühlen, daß sie mittels einer Umwälzpumpe durch eine mit dem Ansatzbehälter verbundene Umwälzleitung mit einem Wärmetauscher umgepumpt wird.

Um Schleimklümpchen abzuscheiden, bevor die Lösung vom Ansatzbehälter in den Druckvorlagebehälter gelangt, ist zwischen beiden eine Filtrationsstrecke angeordnet, die aus einem Grobfilter und einem Feinfilter bestehen kann.

Als vorteilhaft hat sich erwiesen, wenn der Druckvorlagebehälter etwa das 1,5-fache Volumen des Ansatzbehälters besitzt, mit Preßluft oder Stickstoff geregelten Drucks beaufschlagt wird und über ein Steigrohr mit dem Verteilerblock verbunden ist. Wird der Preßluft- bzw. Stickstoffdruck genügend genau eingeregelt, gelangt die Lösung völlig gleichmäßig über den Verteilerblock zu den Düsen.

Vorzugsweise sind die Auffangrinne und der bzw. die Härtungsbehälter über eine Umwälzleitung mit einer Umwälzpumpe miteinander verbunden, so daß ein kontinuierliches Arbeiten unabhängig vom Befüllen und Entleeren der Härtungsbäder möglich ist.

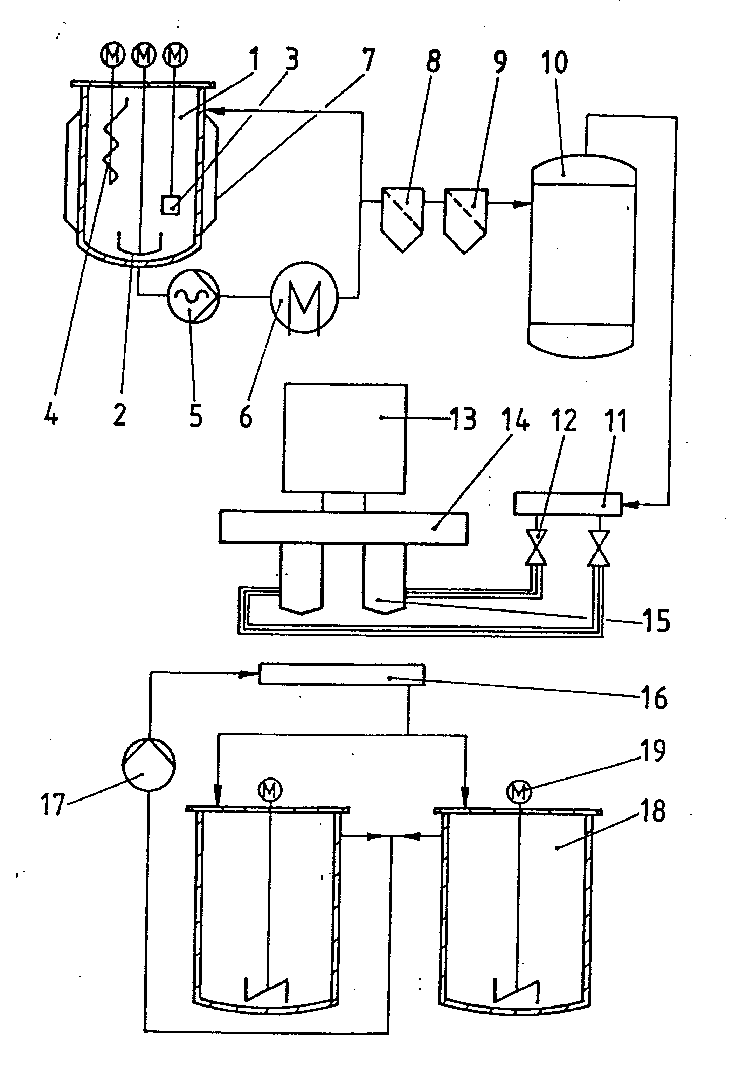

Die Erfindung wird im folgenden anhand eines in der Zeichnung schematisch dargestellten Ausführungsbeispiels näher erläutert.

In einem Ansatzbehälter 1 zum Herstellen einer hochviskosen Alginat-Mikroorganismenlösung befinden sich ein Rührwerk 2, ein Dispergator 3 und ein Heizelement 4. In diesem Ansatzbehälter 1 wird Alginatpulver in Wasser dispergiert, gelöst und zum Abtöten von Keimen und zum Einstellen der Viskosität erhitzt. Das Erhitzen dauert vorzugsweise 15 Minuten bei 120°C oder 45 Minuten bei 90°C; es kann auch durch Beaufschlagen eines Heizmantels 7 mit einer Heizflüssigkeit oder Dampf erfolgen. Wird dem Heizmantel 7 eine Kühlflüssigkeit zugeführt, kühlt die Alginatlösung im Ansatzbehälter 1 wieder ab.

Besonders schnell läßt sich das Abkühlen erreichen, wenn die Lösung durch Umpumpen mittels einer Verdrängerpumpe 5 durch einen Wärmetauscher 6 geleitet wird. Wenn die Lösung auf 30 bis 40°C abgekühlt ist, wird in die Lösung eine Bakterienfeuchtmasse unter Einsatz des Rührwerks 2 eingemischt.

Die die Mikroorganismen, Zellen oder Enzyme enthaltende Lösung wird durch eine Filtrationsstrecke geleitet, um Schleimklümpchen abzuscheiden, bevor die Lösung in einen Druckvorlagebehälter 10 gelangt. Die Alginat-Mikroorganismenlösung wird zunächst durch einen Grobfilter 8 mit einem Lochdurchmesser von 2 mm und anschließend durch einen Feinfilter 9 mit einem Lochdurchmesser von 0,2 mm gedrückt.

Der Druckvorlagenbehälter 10 weist vorzugsweise das anderthalbfache Volumen des Alginatansatzbehälters 1 auf und wird mit Preßluft oder Stickstoff konstanten Drucks in nicht dargestellter Weise beaufschlagt.

Die Lösung verläßt den Druckvorlagebehälter 10 über eine Steigleitung und gelangt zu einem Verteilerblock 11. Dem Verteilerblock 11 sind für jede Düse 15 je ein Dosierventil 12 nachgeordnet, so daß jeder Düse 15 die gleiche Menge Polymerlösung zugeführt wird. Die Zeichnung zeigt nur je zwei der Düsen 15 und Dosierventile 12. Die Düsen 15 sind fest mit einem Düsenträger 14 verbunden und werden durch einen Vibrator 13 über den Düsenträger 14 in Schwingungen versetzt. Die Frequenz und die Amplitude der Schwingung lassen sich einstellen. Als besonders günstig hat sich eine Frequenz von 300 bis 600 Schwingungen je Sekunde erwiesen. Mit dem Düsenträger 14 lassen sich vorzugsweise 10 bis 20 Düsen 15 koppeln, so daß sich ein hoher Durchsatz ergibt. Als besonders wirksam haben sich kegelförmige Düsen 15 mit einem Durchmesser von 0,1 bis 1,5 mm mit scharf geschliffener Kante erwiesen. Zu den Düsen 15 führen Zuleitungen, die wenigstens teilweise so flexibel sind, daß sie die Vibrationen nicht nachteilig beeinflussen.

Infolge der Vibrationen wird jeder aus einer Düse 15 austretende Strahl in einzelne Tropfen zerlegt, die sich in kurzer Entfernung zur Strahlaustrittsstelle zu Perlen mit definiertem Durchmesser zusammenziehen. Die Größe und die Form, sowie die Anzahl der Perlen je Zeiteinheit läßt sich über die Frequenz und die Amplitude des Vibrators 13, den Gasdruck im Druckvorlagebehälter 10 und die Dosierventile 12 so fein einstellen, daß eine Anpassung an vorgegebene Werte in weiten Grenzen möglich ist.

Die Kugeln fallen in eine mit Härtungslösung gefüllte Auffangrinne 15 und werden von dort in Härtungsbehälter 18 gespült. Zu diesem Zweck ist eine Umwälzpumpe 17 so angeordnet, daß Härtungslösung aus dem Härtungsbehälter 18 in die Auffangrinne 16 gepumpt wird und von dort wieder in die Härtungsbehälter 18 zurückfließt. Die Fließgeschwindigkeit in der Auffangrinne 16 ist so gewählt, daß die Perlen in der Rinne weder sich absetzen noch verkleben. Die Tiefe der Auffangrinne 16 ist so bemessen, daß die Perlen nicht auf den Rinnenboden treffen.

Die in die Härtungsbehälter 18 eingespülten Perlen werden dort durch ein nicht dargestelltes Sieb zurückgehalten und mittels eines Rührwerks 19 in der Schwebe gehalten, bis sie ausgehärtet sind.

Über nicht dargestellte Ventile läßt sich jeweils ein Härtungsbehälter 18 zuschalten, während ein anderer Härtungsbehälter 18 entleert wird.

Da die Zeichnung nur eine vereinfachte Darstellung der Vorrichtung ist, sind zweckmäßige Absperrventile z. B. am Ansatzbehälter und weitere Einrichtungen z. B. zur Überwachung von Druck und Temperatur nicht angegeben. Ferner kann die Vorrichtung im Rahmen der Erfindung dadurch abgewandelt werden, daß die Dosierventile 12 in unmittelbarer Nähe der Düsen 15 auf dem Düsenträger 14 angeordnet sind.

## Ansprüche

1. Verfahren zum Herstellen von Biokatalysatoren umfassenden Perlen gleichen Durchmessers aus Perlen bildenden Lösungen mit im Innern der Perlen immobilisierten Zellen oder Enzymen, bei dem eine die Zellen oder Enzyme enthaltende Lösung durch Düsen gedrückt, der aus den Düsen austretende Strahl mittels Vibrationen in Tropfen zerlegt wird und die Tropfen in eine Härtungslösung fallen, dadurch gekennzeichnet, daß die Perlen bildende Lösung einer Vielzahl von gemeinsam an einem mit einem Vibrator gekoppelten Düsenträger angeordneten Düsen gleichzeitig, jedoch für jede Düse einzeln dosierbar zugeführt wird.

2. Verfahren nach Anspruch 1, dadurch gekennzeichnet, daß der Durchmesser der Perlen durch Verändern der Vibratorfrequenz eingestellt wird.

3. Verfahren nach Anspruch 1 oder 2, dadurch gekennzeichnet, daß der Durchmesser der Perlen durch Verändern der Vibratoramplitude eingestellt wird.

4. Verfahren nach einem oder mehreren der Ansprüche 1 bis 3, dadurch gekennzeichnet, daß der Durchmesser der Perlen durch Verändern des Drucks der Lösung eingestellt wird.

5. Verfahren nach einem oder mehreren der Ansprüche 1 bis 4, dadurch gekennzeichnet, daß der Durchmesser der Perlen durch Verändern des Durchflusses durch Dosierventile eingestellt wird.

6. Vorrichtung zur Durchführung des Verfahrens nach einem oder mehreren der Ansprüche 1 bis 5, gekennzeichnet durch wenigstens einen Druckvorlagebehälter (10) für die Perlen bildende Lösung, einen nachgeschalteten Verteilerblock (11), Feindosierventile (12), einen Düsenträger (14) mit mehreren Düsen (15), einen starr mit dem Düsenträger (15) gekoppelten Vibrator (13 und wenigstens einem Härtungsbehälter (18).

7. Vorrichtung nach Anspruch 6, dadurch gekennzeichnet, daß dem Druckvorlagebehälter (10) eine Filtrationsstrecke (8, 9) und wenigstens ein Ansatzbehälter (1) vorgeschaltet sind und unterhalb der Düsen (15) eine die Härtungslösung enthaltende und mit einem Härtungsbehälter (18) verbundene Auffangrinne (16) angeordnet ist.

8. Vorrichtung nach Anspruch 6 oder 7, gekennzeichnet durch kegelförmige Düsen (15) mit scharfer geschliffener Kante und einem Durchmesser von 0,1 bis 1,5 mm.

9. Vorrichtung nach einem oder mehreren der Ansprüche 7 oder 8, dadurch gekennzeichnet, daß der Ansatzbehälter (1) mit einem Rührwerk (2) und einem Dispergator (3) sowie Einrichtungen zum Temperieren der Lösung insbesondere in Form einer mit dem Ansatzbehälter (1) verbundenen Umwälzleitung mit einer Umwälzpumpe (5) und einem Wärmetauscher (6) versehen ist.

10. Vorrichtung nach einem oder mehreren der Ansprüche 7 bis 9, dadurch gekennzeichnet, daß die Auffangrinne (16) und der Härtungsbehälter (18) über eine Umwälzleitung mit einer Umwälzpumpe (17) in Verbindung stehen, wobei eine Umwälzung von aushärtenden Perlen durch die Umwälzleitung durch ein Sieb verhindert wird.